# EUROPÄISCHE PATENTSCHRIFT

(11) **EP 2 656 049 B1**
(45) Veröffentlichungstag und Bekanntmachung des Hinweises auf die Patenterteilung: **05.10.2016**
(21) Anmeldenummer: 11808582.8
(22) Anmeldetag: 16.12.2011
(51) Int. Cl.: G01N 21/3504, C12M 1/00, C12M 1/34

(54) **MESSYSTEM ZUM MESSEN DER CO2-KONZENTRATION IN EINEM KLIMASCHRANK ODER EINEM INKUBATOR**
MEASURING SYSTEM FOR MEASURING THE CO2-CENCENTRATION IN AN INCUBATOR
SYSTÈME DE MESURE POUR MESURER LA CONCENTRATION DE CO2 DANS UN INCUBATEUR

(30) Priorität: 20.12.2010 DE 102010055182
(43) Veröffentlichungstag der Anmeldung: 30.10.2013
(73) Patentinhaber: Binder GmbH, 78532 Tuttlingen (DE)
(72) Erfinder: BASCHANT, Dieter, 78166 Geisingen/Pfohren (DE); BINDER, Peter, Michael, 78532 Tuttlingen (DE); VON BOTH, Holger, 78234 Engen (DE); MAGI, André, 07619 Mertendorf (DE); SACHSE, Patrick, 07586 Kraftsdorf (DE); BIERMANN, Steffen, 07629 Hermsdorf (DE)
(74) Vertreter: Westphal, Mussgnug & Partner Patentanwälte mbB
(86) Internationale Anmeldenummer: PCT/EP2011/006376
(87) Internationale Veröffentlichungsnummer: WO 2012/084162

(56) Entgegenhaltungen:
- EP-A1- 0 670 486
- EP-A1- 1 564 545
- EP-A2- 0 826 956
- WO-A1-96/01418
- GB-A- 740 374
- GB-A- 2 317 010
- US-A1- 2002 153 490

## Beschreibung

Die Erfindung betrifft ein Messsystem zum Messen der CO₂-Konzentration in einem Klimaschrank oder einem Inkubator.

Bekannt sind Messsysteme zum Messen der CO₂-Konzentration, welche eine Strahlungsquelle und wenigstens zwei Strahlungsdetektoren aufweisen, wobei die Strahlungsquelle und die Strahlungsdetektoren derart zueinander angeordnet sind, dass die von der Strahlungsquelle ausgesandte Strahlung nach Durchquerung eines Messvolumens auf die Strahlungsdetektoren trifft.

Darüber hinaus sind aus der EP 0 670 486 A1, der WO 96/01418 und der GB 2 317 010 A auch Messsysteme mit einer Strahlungsquelle mit einer optischen Achse und wenigstens zwei Strahlungsdetektoren bekannt, bei denen Strahlungsquelle und Strahlungsdetektoren derart zueinander angeordnet sind, dass die von der Strahlungsquelle ausgesandte Strahlung nach Durchquerung eines Messvolumens auf die Strahlungsdetektoren trifft, wobei für jeden Strahlungsdetektor jeweils ein Kanal mit einem ersten Ende und einem zweiten Ende vorgesehen ist, wobei der Strahlungsdetektor an dem ersten Ende angeordnet ist und das erste Ende des Kanals von der Strahlungsquelle abgewandt ist und wobei die Kanäle einen Durchmesser aufweisen, welcher kleiner ist als der Durchmesser des Messvolumens quer zur optischen Achse.

Wünschenswert ist es, ein Messsystem zum Messen der CO₂-Konzentration bereit zu stellen, welches auch in Klimaschränken oder Inkubatoren verwendet werden kann und somit auch bei Temperaturen, bei welchen eine Sterilisation erfolgt, d. h. insbesondere bei Temperaturen von 180°, nicht beschädigt wird.

Viele bekannte Messsysteme zum Messen der CO₂-Konzentration sind nicht temperaturstabil.

Die Aufgabe der Erfindung besteht daher darin, ein Messsystem zum Messen der CO₂-Konzentration bereit zu stellen, welches in Klimaschränken oder Inkubatoren verwendet werden kann.

Die Aufgabe der Erfindung wird gelöst durch ein Messsystem zum Messen der CO₂-Konzentration mit den Merkmalen des Patentanspruchs 1.

Vorteilhafte Ausgestaltungen und Weiterbildungen der Erfindung sind in den Unteransprüchen angegeben.

Das erfindungsgemäße Messsystem zum Messen der CO₂-Konzentration in einem Klimaschrank oder einem Inkubator weist eine Strahlungsquelle mit einer optischen Achse und wenigstens zwei, vorzugsweise drei, Strahlungsdetektoren auf, wobei die Strahlungsquelle und die Strahlungsdetektoren derart zueinander angeordnet sind, dass die von der Strahlungsquelle ausgesandte Strahlung nach Durchquerung eines Messvolumens auf die Strahlungsdetektoren trifft, und zeichnet sich dadurch aus, dass für jeden Strahlungsdetektor jeweils ein Kanal mit einem ersten Ende und einem zweiten Ende vorgesehen ist, wobei der Strahlungsdetektor an dem ersten Ende angeordnet ist und das erste Ende des Kanals von der Strahlungsquelle abgewandt ist, und wobei die Kanäle einen Durchmesser aufweisen, welcher kleiner ist als der Durchmesser des Messvolumens quer zur optischen Achse.

Das Licht, das bei dieser Anordnung auf die Strahlungsdetektoren trifft, muss somit zuerst den zu dem Strahlungsdetektor gehörigen Kanal durchqueren. Dabei wirkt das der Strahlungsquelle zugewandte Ende des Kanals dadurch, dass die Kanäle einen Durchmesser aufweisen, welcher kleiner ist als der Durchmesser des Messvolumens, wie eine Blende und verringert somit den Eintritt von Streulicht in den entsprechenden Kanal. Zudem bewirkt die Längserstreckung des Kanals, dass durch das erste Ende in den Kanal eingetretenes Streulicht in der Regel nicht auf den am Ende des Kanals angeordneten Strahlungsdetektor fallen kann. Insgesamt wird durch die erfindungsgemäße Anordnung ein Einfall von reflektierter Strahlung auf die Strahlungsdetektoren so gut wie ausgeschlossen. Damit eignet sich das Messsystem insbesondere zum Einsatz in Klimaschränken oder Inkubatoren, wo es starken Temperaturschwankungen ausgesetzt ist.

Gemäß einer bevorzugten Ausführungsform der Erfindung weisen die Kanäle eine Achse auf, wobei die Achse wenigstens eines der Kanäle, vorzugsweise aller Kanäle, gegen die optische Achse in einem Winkel geneigt angeordnet ist. Auf diese Weise kann ein direkter Strahlengang zwischen der Strahlungsquelle und dem Strahlungsdetektor für mehrere Strahlungsdetektoren erreicht werden.

Vorteilhafterweise beträgt das Verhältnis von Abstand zwischen der Strahlungsquelle und einem der Strahlungsdetektoren zu Länge des zu dem Strahlungsdetektor gehörigen Kanals etwa 4:1. Durch diese Konstruktion kann ein Einfall von reflektierter Strahlung auf den Strahlungsdetektor zuverlässig verringert werden.

Gemäß einer bevorzugten Ausführungsform der Erfindung weisen die Strahlungsdetektoren eine strahlungsempfindliche Fläche auf, welche tangential zu einer Kugeloberfläche angeordnet ist. Durch diese Anordnung kann ein im Wesentlichen senkrechter Einfall der von der Strahlungsquelle ausgesandten Strahlung auf alle Strahlungsdetektoren ermöglicht werden.

Vorzugsweise ist die Strahlungsquelle als Infrarotstrahlungsquelle, insbesondere als thermische Strahlungsquelle, ausgebildet, welche sich für die Anwendung in Messsystemen zur Messung der CO₂-Konzentration besonders eignen.

Die Strahlungsquelle und die Strahlungsdetektoren sind in einem Gehäuse angeordnet, welches das Messvolumen umschließt und welches ein Bodenteil, ein Deckelteil und ein zwischen dem Bodenteil und dem Deckelteil angeordnetes Mittelteil aufweist, wobei vorzugsweise die Strahlungsquelle in dem Bodenteil und/oder die Strahlungsdetektoren in dem Deckelteil angeordnet sind. Auf diese Weise wird ein einfacher und kostengünstiger Aufbau des Messsystems ermöglicht.

Vorzugsweise ist das Mittelteil aus Kunststoff gefertigt, was eine kostengünstige Herstellung sowie eine thermische Entkopplung zwischen Strahlerelement und Infrarotdetektor ermöglicht.

Die Innenseite des Mittelteils weist eine strukturierte Oberfläche, Rippen oder ein Gewinde, insbesondere ein Feingewinde, auf, um auf diese Weise Reflektionen an der Innenseite des Mittelteils möglichst weitgehend zu unterbinden.

Vorzugsweise sind die Kanäle in dem Deckelteil angeordnet, was eine einfache und kostengünstige Herstellung ermöglicht.

Gemäß einer bevorzugten Ausführungsform ist das Deckelteil aus Aluminium gefertigt, dessen Oberfläche vorzugsweise eloxiert ist. Durch die Fertigung aus Aluminium wird es ermöglicht, die Strahlungsdetektoren auf einer gewünschten Temperatur zu halten und die anfallende Wärme abzuführen. Durch die Verwendung von eloxiertem Aluminium werden Reflektionen an dem Deckelteil, insbesondere an den Kanalinnenwänden, weitgehend vermieden.

Die Erfindung wird anhand der nachfolgenden Figuren ausführlich erläutert. Es zeigt
- Fig. 1: eine perspektivische Ansicht eines erfindungsgemäßen Messsystems,
- Fig. 2: eine Seitenansicht des Messsystems gemäß Figur 1,
- Fig. 3: einen Längsschnitt durch das Messsystem gemäß Figur 1,
- Fig. 4: eine Draufsicht auf das Messsystem gemäß Figur 1,
- Fig. 5: eine Ausschnittsvergrößerung aus Figur 3,
- Fig. 6: eine perspektivische Ansicht eines Deckelteils des Messsystems gemäß Figur 1,
- Fig. 7: eine Seitenansicht des Deckelteils gemäß Figur 6,
- Fig. 8: eine Draufsicht auf das Deckelteil gemäß Figur 6,
- Fig. 9: eine perspektivische Ansicht eines weiteren Ausführungsbeispiels eines Deckelteils,
- Fig. 10: eine weitere perspektivische Ansicht des Deckelteils gemäß Figur 9 und
- Fig. 11: eine Draufsicht auf das Deckelteil gemäß Figur 9.

Gleiche Bezugsziffern bezeichnen gleiche oder funktionsgleiche Teile, wobei zur besseren Übersicht nicht in sämtlichen Figuren sämtliche Bezugsziffern angegeben sind.

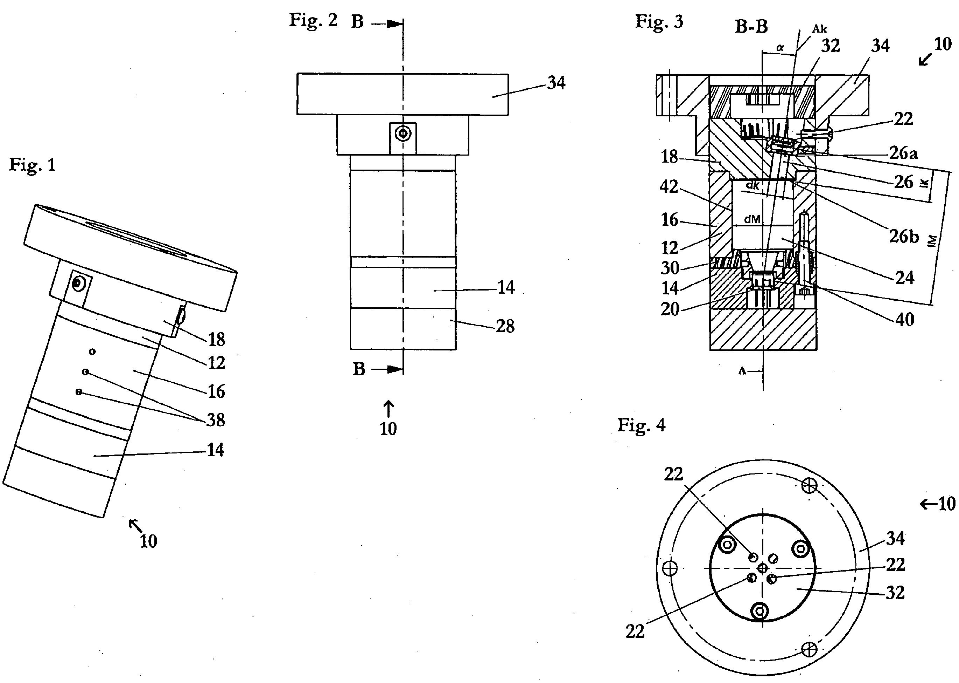

Die Figuren 1 bis 5 zeigen verschiedene Ansichten eines Messsystems 10 zum Messen der CO₂-Konzentration in einem Klimaschrank oder einem Inkubator. Das Messsystem 10 weist ein Gehäuse 12 auf, welches ein Bodenteil 14, ein Mittelteil 16 und ein Deckelteil 18 aufweist. Das Mittelteil 16 ist zwischen dem Bodenteil 14 und dem Deckelteil 18 angeordnet und vorzugsweise rohrförmig ausgebildet. Das Gehäuse 12 umschließt das Messvolumen 24, wobei in dem Mittelteil 16 und/oder dem Deckelteil 18 eine oder mehrere Gasdurchtrittsöffnungen 38 angeordnet sind, über welche das zu untersuchende Gas in das Messvolumen 24 des Messsystems 10 eintreten kann.

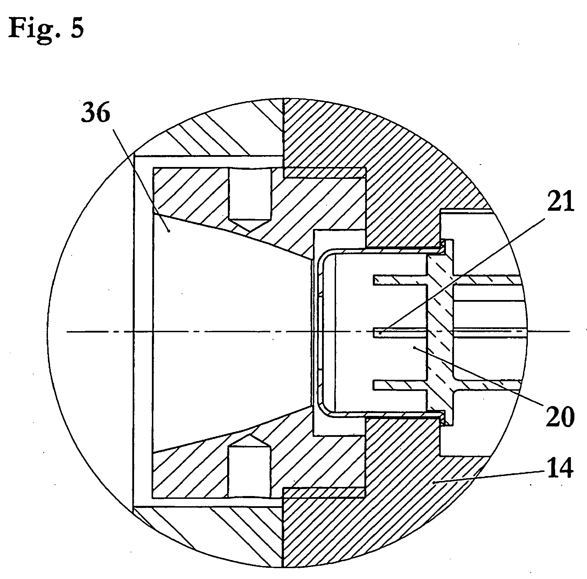

Das Messsystem 10 weist eine Strahlungsquelle 20 und drei Strahlungsdetektoren 22 auf. Die Strahlungsquelle 20 (vgl. insbesondere Fig. 5)ist in dem Bodenteil 14 angeordnet und beispielsweise als Infrarotstrahlungsquelle, insbesondere als thermische Strahlungsquelle, ausgebildet. Die Strahlungsquelle 20 weist daher insbesondere eine Strahlerfläche 21 auf, vor welcher ein Reflektorelement 36 zur Bündelung der ausgesandten Strahlung angeordnet ist. Insbesondere weist die Strahlungsquelle 20 eine optische Achse A auf.

Das Bodenteil 14 ist mit Hilfe von Schrauben 40 an dem Mittelteil 16 verschraubt, wobei vorzugsweise zwischen dem Bodenteil 14 und dem Mittelteil 16 ein Isolierelement 30 angeordnet ist, welches der Wärmeisolation dient. An dem Bodenteil 14 ist vorzugsweise ein Kühlkörper 28 angeordnet, welcher beispielsweise Kühlrippen aufweist und der Kühlung des Bodenteils 14 dient.

Jedem der Strahlungsdetektoren 22 ist ein Kanal 26 zugeordnet, wobei jeder der Kanäle 26 ein erstes Ende 26a und ein zweites Ende 26b aufweist. Der Strahlungsdetektor 22 ist dabei an dem ersten Ende 26a des Kanals 26 angeordnet, wobei der Kanal 26 derart ausgerichtet ist, dass das erste Ende 26a des Kanals 26 von der Strahlungsquelle 20 abgewandt ist, während das zweite Ende 26b des Kanals 26 der Strahlungsquelle 20 zugewandt ist. Jeder der Kanäle 26 weist eine Achse AK auf. Die Kanäle 26 sind insbesondere in dem Deckelteil 18 angeordnet und insbesondere als Durchgangsöffnungen durch das Deckelteil 18 ausgebildet.

Die Strahlungsdetektoren 22 weisen eine strahlungsempfindliche Fläche auf, welche im Wesentlichen tangential an einer Kugeloberfläche um die Strahlungsquelle 20 angeordnet sind. Auf diese Weise wird ermöglicht, dass von der Strahlungsquelle 20 ausgesandtes Licht im Wesentlichen senkrecht auf sämtliche Strahlungsdetektoren 22 auftrifft. Weiterhin sind die Strahlungsdetektoren 22 vorzugsweise an den Ecken eines gleichseitigen Dreiecks angeordnet, um einen gleichmäßigen Strahlungseinfall auf alle Strahlungsdetektoren 22 zu ermöglichen. Damit der direkte Strahlungsgang zwischen der Strahlungsquelle 20 und dem jeweiligen Strahlungsdetektor 22 nicht durch die Kanäle 26 beeinträchtigt wird, ist die Achse AK der Kanäle 26 gegen die optische Achse A in einem Winkel α geneigt angeordnet. Der Winkel a beträgt dabei etwa 10°.

Die Kanäle 26 verhindern jedoch den Einfall von Streulicht auf die Strahlungsdetektoren 22. Die Kanäle 26 weisen einen Durchmesser dK auf, welcher geringer ist als ein Durchmesser dM des Mittelteils 16 und insbesondere als der Durchmesser dM des Mittelteils des Messvolumens 24 quer zur optischen Achse A. Auf diese Weise wird durch das zweite Ende 26b des Kanals 26 eine Blendenwirkung erreicht, wodurch der Streulichteinfall verringert wird. Der Streulichteinfall wird weiterhin verringert durch die Längserstreckung der Kanäle 26. Besonders günstig zur Vermeidung von Streulichteinfall ist ein Verhältnis der Länge 1K des Kanals 26 zu dem Abstand 1M zwischen der Strahlungsquelle 20 und einem der Strahlungsdetektoren 22 von 1:4 (vgl. insbesondere Fig. 3).

Um Strahlungsreflektionen im Inneren des Gehäuses 12 weiter zu verringern, weist das Mittelteil 16 auf der Innenfläche vorzugsweise eine strukturierte Oberfläche, beispielsweise in Form eines Feingewindes 42, auf. Das Mittelteil 16 ist insbesondere aus Kunststoff gefertigt, da Kunststoff ein reflektionsarmes Material ist, das die thermische Entkopplung zwischen Strahler und Detektor gewährleistet, und sich kostengünstig verarbeiten lässt. Das Deckteil 18 ist vorzugsweise aus Aluminium gefertigt, um die in den Strahlungsdetektoren anfallende Wärme 22 besser abführen zu können. Um Reflektionen insbesondere an den Innenseiten der Kanäle 26 zu vermeiden, ist das Deckelteil 18 vorzugsweise aus eloxiertem Aluminium gefertigt.

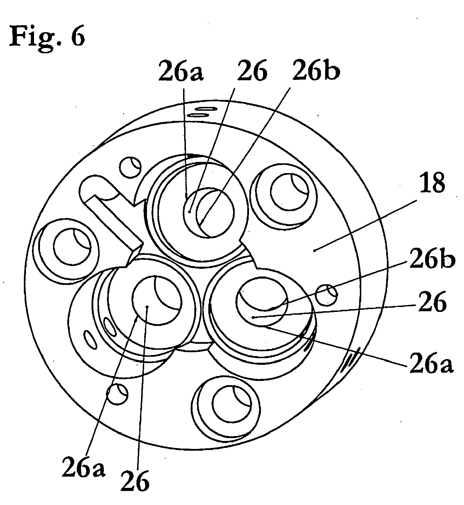

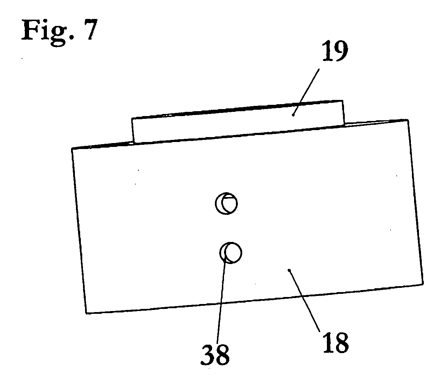

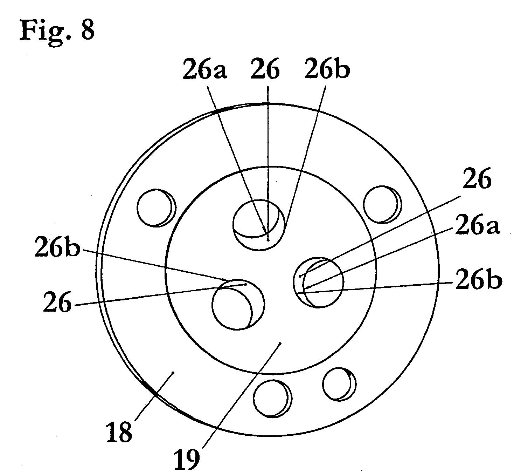

Die Figuren 6 bis 8 zeigen weitere Ansichten des Deckelteils 18. Das Deckelteil 18 ist im Wesentlichen zylindrisch ausgebildet und weist an der dem Mittelteil 16 zugewandten Stirnfläche einen zylindrischen Vorsprung 19 kleineren Durchmessers auf, welcher formschlüssig in das Mittelteil 16 eingesetzt werden kann. Das Deckelteil 18 kann mittels nicht dargestellter Schrauben an dem Mittelteil 16 verschraubt werden. Die dem Messvolumen 24 abgewandte Stirnfläche des Deckelteils 18 kann durch ein Isolierelement 32 abgedeckt werden. Zudem kann an der dem Messvolumen 24 abgewandten Seite des Deckelteils 18 ein Montageflansch 34 zur Montage des Messsystems 10 in einem Klimaschrank oder einem Inkubator angeordnet werden.

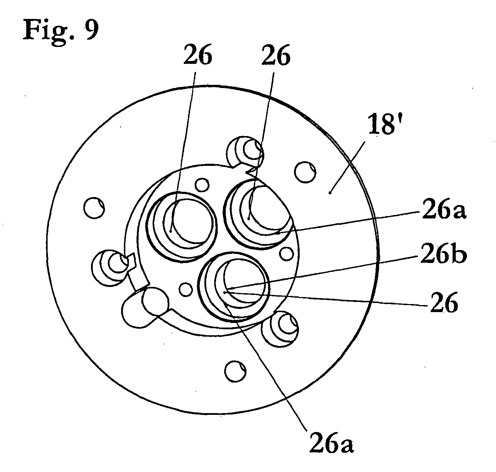

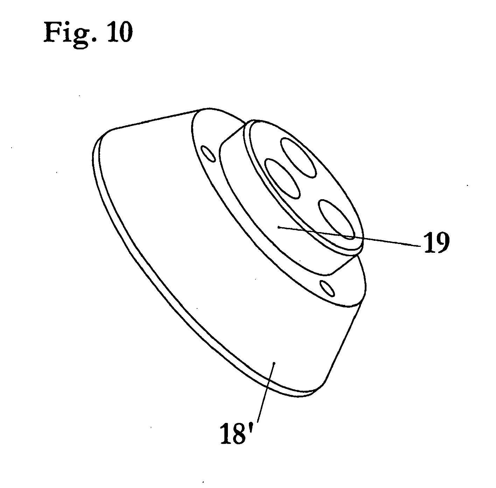

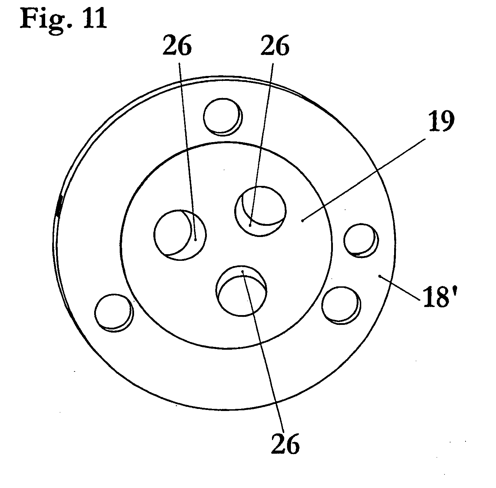

Die Figuren 9 bis 11 zeigen verschiedene Ansichten eines alternativen Ausführungsbeispiels eines Deckelteils 18', welches sich von dem Deckelteil 18 gemäß der Figuren 6 bis 8 lediglich dadurch unterscheidet, dass das Deckelteil 18' nicht zylindrisch, sondern konisch zulaufend ausgebildet ist.

### Bezugszeichenliste

- 10: Messsystem
- 12: Gehäuse
- 14: Bodenteil
- 16: Mittelteil
- 18: Deckelteil
- 18': Deckelteil
- 19: Vorsprung
- 20: Strahlungsquelle
- 21: Strahlenfläche
- 22: Strahlungsdetektoren
- 24: Messvolumen
- 26: Kanal
- 26a: erstes Ende
- 26b: zweites Ende
- 28: Kühlkörper
- 30: Isolierelement
- 32: Isolierelement
- 34: Montageflansch
- 36: Reflektorelement
- 38: Gasdurchtrittsöffnungen
- 40: Schrauben
- 42: Feingewinde

- α: Winkel
- A: optische Achse
- AK: Achse
- dK: Durchmesser Kanal
- dm: Durchmesser Messvolumen
- 1K: Länge Kanal
- 1M: Abstand

## Patentansprüche

1. Messsystem (10) zum Messen der CO₂-Konzentration in einem Klimaschrank oder einem Inkubator mit einer Strahlungsquelle (20) mit einer optischen Achse (A) und wenigstens zwei, vorzugsweise drei, Strahlungsdetektoren (22), wobei die Strahlungsquelle (20) und die Strahlungsdetektoren (20) derart zueinander angeordnet sind, dass die von der Strahlungsquelle ausgesandte Strahlung nach Durchquerung eines Messvolumen (24) auf die Strahlungsdetektoren (22) trifft, wobei für jeden Strahlungsdetektor (22) jeweils ein Kanal (26) mit einem ersten Ende (26a) und einem zweiten Ende (26b) vorgesehen ist, wobei der Strahlungsdetektor (22) an dem ersten Ende (26a) angeordnet ist und das erste Ende (26a) des Kanals (26) von der Strahlungsquelle abgewandt ist, und wobei die Kanäle einen Durchmesser (dK) aufweisen, welcher kleiner ist als der Durchmesser (dM) des Messvolumens (24) quer zur optischen Achse (4), wobei die Strahlungsquelle (20) und die Strahlungsdetektoren (22) in einem Gehäuse (12) angeordnet sind, welches das Messvolumen (24) umschließt und welches ein Bodenteil (14), ein Deckelteil (18, 18') und ein zwischen dem Bodenteil (14) und dem Deckelteil (18, 18') angeordnetes Mittelteil (16) aufweist,
**dadurch gekennzeichnet, dass** die Innenseite des Mittelteils (16) eine strukturierte Oberfläche, Rippen oder ein Gewinde (42) aufweist.

2. Messsystem nach Anspruch 1,
**dadurch gekennzeichnet, dass** die Kanäle (26) eine Achse (AK)aufweisen, wobei die Achse (AK) wenigstens eines der Kanäle (26), vorzugsweise aller Kanäle (26), gegen die optische Achse (A) in einem Winkel (α) geneigt angeordnet ist.

3. Messsystem nach einem der vorhergehenden Ansprüche,
**dadurch gekennzeichnet, dass** das Verhältnis von Abstand (lM) zwischen der Strahlungsquelle (20) und einem der Strahlungsdetektoren (22) zu Länge (lK) des zu dem Strahlungsdetektor (22) gehörigen Kanals (26) etwa 4:1 beträgt.

4. Messsystem nach einem der vorhergehenden Ansprüche,
**dadurch gekennzeichnet, dass** die Strahlungsdetektoren (22) eine strahlungsempfindliche Fläche aufweisen, welche tangential zu einer Kugeloberfläche angeordnet ist.

5. Messsystem nach einem der vorhergehenden Ansprüche,
**dadurch gekennzeichnet, dass** die Strahlungsquelle (20) als Infrarotstrahlungsquelle, insbesondere als thermische Strahlungsquelle, ausgebildet ist.

6. Messsystem nach einem der vorhergehenden Ansprüche,
**dadurch gekennzeichnet, dass** die Strahlungsquelle (20) in dem Bodenteil (14) und/oder die Strahlungsdetektoren (22) in dem Deckelteil (18, 18'), angeordnet sind.

7. Messsystem nach einem der vorstehenden Ansprüche,
**dadurch gekennzeichnet, dass** das Mittelteil (16) aus Kunststoff gefertigt ist.

8. Messsystem nach einem der Ansprüche 1 bis 7,
**dadurch gekennzeichnet, dass** die Kanäle (26) in dem Deckelteil (18, 18') angeordnet sind.

9. Messsystem nach einem der Ansprüche 1 bis 8,
**dadurch gekennzeichnet, dass** das Deckelteil (18, 18') aus Aluminium gefertigt ist, dessen Oberfläche vorzugsweise eloxiert ist.

## Claims

1. Measurement system (10) for measuring the CO₂ concentration in a climatic cabinet or an incubator, having a radiation source (20) having an optical axis (A) and at least two, preferably three, radiation detectors (22), wherein the radiation source (20) and the radiation detectors (20) are arranged with respect to one another in such a way that the radiation emitted by the radiation source hits the radiation detectors (22) after crossing a measurement volume (24), wherein a channel (26) is provided with a first end (26a) and a second end (26b) for each radiation detector (22) respectively, wherein the radiation detector (22) is arranged on the first end (26a) and the first end (26a) of the channel (26) is facing away from the radiation source, and wherein the channels have a diameter (dK) which is smaller than the diameter (dM) of the measurement volume (24) transversely to the optical axis (4), wherein the radiation source (20) and the radiation detectors (22) are arranged in a housing (12) which surrounds the measurement volume (24) and which has a base part (14), a cover part (18, 18') and a central part (16) arranged between the base part (14) and the cover part (18, 18'),
**characterised in that** the inner side of the central part (16) has a structured surface, ribs or a thread (42).

2. Measurement system according to claim 1,
**characterised in that** the channels (26) have an axis (AK), wherein the axis (AK) of at least one of the channels (26), preferably of all channels (26), is arranged with inclination relative to the optical axis (A) at an angle (α).

3. Measurement system according to one of the preceding claims,
**characterised in that** the ratio of distance (1M) between the radiation source (20) and one of the radiation detectors (22) with regard to the length (lK) of the channel (26) belonging to the radiation detector (22) is approximately 4:1.

4. Measurement system according to one of the preceding claims,
**characterised in that** the radiation detectors (22) have a radiation-sensitive surface which is arranged tangentially to a spherical surface.

5. Measurement system according to one of the preceding claims,
**characterised in that** the radiation source (20) is formed as an infrared radiation source, in particular as a thermal radiation source.

6. Measurement system according to one of the preceding claims,
**characterised in that** the radiation source (20) is arranged in the base part (14) and/or the radiation detectors (22) are arranged in the cover part (18, 18').

7. Measurement system according to one of the preceding claims,
**characterised in that** the central part (16) is produced from plastic.

8. Measurement system according to one of claims 1 to 7,
**characterised in that** the channels (26) are arranged in the cover part (18, 18').

9. Measurement system according to one of claims 1 to 8,
**characterised in that** the cover part (18, 18') is produced from aluminium, the surface of which is preferably anodised.

## Revendications

1. Système de mesure (10) destiné à permettre de mesurer la concentration en CO₂ dans une armoire de climatisation ou un incubateur comprenant une source de rayonnement (20) ayant un axe optique (A) et au moins deux et de préférence trois détecteurs de rayonnement (22), la source de rayonnement (20) et les détecteurs de rayonnement (22) étant montés les uns par rapport aux autres de sorte que après avoir traversé un volume de mesure (24) le rayonnement émis par la source de rayonnement viennent frapper, les détecteurs de rayonnement (22), pour chacun des détecteurs de rayonnement (22) étant respectivement prévu un canal (26) ayant une première extrémité (26a) et une seconde extrémité (26b), le détecteur de rayonnement (22) étant monté à la première extrémité (26a), et la première extrémité (26a) du canal (26) étant située à l'opposé de la source de rayonnement, et les canaux ayant un diamètre (dK) qui est inférieur au diamètre (dM) du volume de mesure transversalement à l'axe optique (4), la source de rayonnement (20) et les détecteurs de rayonnement (22) étant montés dans un boîtier (12) qui entoure le volume de mesure (24) et comporte une partie de fond (14), une partie de recouvrement (18, 18') et une partie médiane (16) située entre la partie de fond (14) et la partie de recouvrement (18, 18'), **caractérisé en ce que**
la face interne de la partie médiane (16) a une surface structurée, des nervures ou un filetage (42).

2. Système de mesure conforme à la revendication 1,
**caractérisé en ce que**
les canaux (26) ont un axe (AK), l'axe (AK) d'au moins l'un des canaux (26) et de préférence de tous les canaux (26) étant inclinés d'un angle (α) par rapport à l'axe optique (A).

3. Système de mesure conforme à l'une des revendications précédentes,
**caractérisé en ce que**
le rapport entre la distance (IM) entre la source de rayonnement (20) et l'un des détecteurs de rayonnement (22) et la longueur (1K) du canal (26) associé au détecteur de rayonnement (22) est d'environ 4 : 1.

4. Système de mesure conforme à l'une des revendications précédentes,
**caractérisé en ce que**
les détecteurs de rayonnement (22) ont une surface sensible au rayonnement qui est située tangentiellement à une surface sphérique.

5. Système de mesure conforme à l'une des revendications précédentes,
**caractérisé en ce que**
la source de rayonnement (20) est réalisée sous la forme d'une source de rayonnement infrarouge, en particulier sous la forme d'une source de rayonnement thermique.

6. Système de mesure conforme à l'une des revendications précédentes,
**caractérisé en ce que**
la source de rayonnement (20) est située dans la partie de fond (14) et/ou les détecteurs de rayonnement (22) sont situés dans la partie de recouvrement (18, 18').

7. Système de mesure conforme à l'une des revendications précédentes,
**caractérisé en ce que**
la partie médiane (16) est réalisée en matériau synthétique.

8. Système de mesure conforme à l'une des revendications 1 à 7,
**caractérisé en ce que**
les canaux (26) sont situés dans la partie de recouvrement (18, 18').

9. Système de mesure conforme à l'une des revendications 1 à 8,
**caractérisé en ce que**
la partie de recouvrement (18, 18') est réalisée en aluminium et sa surface est de préférence oxydée électrolytiquement.
